# EUROPEAN PATENT SPECIFICATION

(11) **EP 2 349 470 B1**
(45) Date of publication and mention of the grant of the patent: **04.06.2014**
(21) Application number: 09792483.1
(22) Date of filing: 11.09.2009
(51) Int. Cl.: A61B 5/08, A61N 1/365

(54) **PACEMAKER WITH NEUROCARDIOGENIC SYNCOPE DETECTION AND THERAPY UTILIZING MINUTE VENTILATION INPUT**
SCHRITTMACHER MIT NEUROKARDIOGENEM SYNKOPE-NACHWEIS UND THERAPIE UNTER VERWENDUNG DES MINUTENVENTILATIONSINPUTS
STIMULATEUR CARDIAQUE DOTÉ D'UN DISPOSITIF DE DÉTECTION DE SYNCOPE NEUROCARDIOGÉNIQUE ET TRAITEMENT UTILISANT UNE ENTRÉE DE VENTILATION PRÉCISE

(30) Priority: 12.09.2008 US 96494 P
(43) Date of publication of application: 03.08.2011
(73) Proprietor: LeLorier, Paul, Mandeville LA 70471 (US)
(72) Inventor: LeLorier, Paul, Mandeville LA 70471 (US)
(74) Representative: Emde, Eric
(86) International application number: PCT/US2009/056734
(87) International publication number: WO 2010/030942

(56) References cited:
- US-A- 6 049 735
- US-A1- 2003 139 780
- ARVINDER S. KURBAAN, MARK ERICKSON, MARK E.V. PETERSEN, ANN-CHRISTINE FRANZEN, ZARA STACK, TIMOTHY WILLIAMS: "Respiratory Changes in Vasovagal Syncope" JOURNAL OF CARDIOVASCULAR ELECTROPHYSIOLOGY, vol. 11, no. 6, 20 April 2007 (2007-04-20) , pages 607-611, XP002561622

## Description

### BACKGROUND

### 1. Field

The subject invention relates to an implantable medical device, such as a pacemaker, with neurocardiogenic syncope detection and therapy utilizing minute ventilation input.

### 2. Related Art

Neurocardiogenic syncope (vasovagal syncope, the common "fainting spell") is a common but complex physiologic disorder. The condition is marked by a sudden drop in heart rate and blood pressure, resulting in decreased cerebral perfusion and subsequent loss of consciousness and postural tone. This condition is unpleasant and limiting for the patient as well as potentially dangerous; unexpected episodes of syncope may result in injury from falls. Behavioral treatment for recurrent neurocardiogenic syncope has been limited to lifestyle limitation, avoidance behaviors and abortive maneuvers, and liberalization of fluid and sodium intake. Medical therapy has been limited to selective serotonin reuptake inhibitors and the "off label" use of fludrocortisone, a mineralocorticoid that enhances sodium and water retention. The latter therapy is often unacceptable in older patients with preexisting heart disease or hypertension.

Given the limitations of medical and behavioral therapy, some consideration has been given to the role of cardiac pacemakers in treating neurocardiogenic syncope. Since an abrupt drop in heart rate is a prominent feature of most neurocardiogenic syncopal episodes, prior devices and algorithms have focused on various "rate drop response" algorithms, in which the pacemaker detects when the patient's heart rate drops below a lower hysteresis rate and determines whether the rate of decrease in heart rate (dHR/dT) exceeds a predetermined value; if this condition is met, then the pacemaker output is set to the hysteresis rate for a preset time interval.

Various iterations of rate drop response are known. Initial uncontrolled trials suggested improved outcomes with pacing. However, subsequent blinded trials, in which pacemakers were implanted but not activated in the control group, demonstrated a significant placebo effect and no significant difference between treatment and control groups. As a consequence, the use of pacemakers for the treatment for neurocardiogenic syncope has been called into question. Nonetheless, cardiac pacemakers are frequently implanted for this indication. Furthermore, older patients with neurocardiogenic syncope often have other clinical indications for cardiac pacemakers or internal cardiac defibrillators (ICDs). In their case, the implantation of a cardiac pacemaker or ICD with traditional pacing/defibrillating algorithms may not effectively treat all of their syncopal episodes. A need remains, therefore, for providing a cardiac pacing device that has enhanced detection and treatment of impending neurocardiogenic syncope.

The hemodynamic changes seen during neurocardiogenic syncope may be approximated during head up tilt table testing. Previous investigations have noted that, in addition to decreases in heart rate and blood pressure, changes in breathing patterns may also occur before fainting. Subjects have been observed to yawn, sigh, or hyperventilate before syncope, suggesting that alterations in respiration may accompany sudden changes in autonomic control of the heart and peripheral vasculature.

The causal relationship between respiratory variation and neurocardiogenic syncope has been unclear. Vasomotor instability preceding syncope has been previously discussed; however, early work did not show a convincing relationship between respiration and syncope in healthy volunteers. Studies using indirect measurement of minute ventilation and complex demodulation have demonstrated that hyperpnea precedes increases in cardiac vagal tone and subsequent tilt-induced syncope in healthy volunteers.

US Patent Application US 2003/0 139 780 A1 by Markowitz et al. discloses an implantable medical device for detection and treatment of syncope. The onset of a syncopal episode is declared when the patient's respiration rate and/or tidal volume and/or minute ventilation increases by a predetermined increment.

### SUMMARY

The following summary of the invention is included in order to provide a basic understanding of some aspects and features of the invention. This summary is not an extensive overview of the invention and as such it is not intended to particularly identify key or critical elements of the invention or to delineate the scope of the invention. Its sole purpose is to present some concepts of the invention in a simplified form as a prelude to the more detailed description that is presented below. The invention is defined in claim 1.

A method is disclosed for cardiac pacing that includes detecting a series of intrinsic depolarizations of a heart; detecting minute ventilation and respiratory rate; sampling baseline minute ventilation and respiratory rate; detecting an increase in minute ventilation over a predetermined period of time that satisfies a programmed criteria; detecting any rate of change in respiratory rate; determining whether the change in minute ventilation is a sole function of increased tidal volume; and delivering cardiac pacing therapy if minute ventilation criteria are met at a pacing rate above the intrinsic heart rate for a predetermined period of time.

The increase in minute ventilation may be determined to be the result of increased tidal volume, with a relatively fixed respiratory rate.

The determination of baseline minute ventilation and its components, tidal volume and respiratory rate may be determined by measurement of transthoracic impedance.

The increase in tidal volume may be defined as being more than a 75% increase from baseline.

The fixed respiratory rate may be defined as a less than 25% variance in respiratory period, R.

The method also includes storing at least one data item related to the step of detecting an increase in minute ventilation.

A computer readable medium for storing instructions for performing a method is disclosed that includes instructions for detecting a series of intrinsic depolarizations of a heart; detecting minute ventilation and respiratory rate; sampling baseline minute ventilation and respiratory rate; detecting an increase in minute ventilation over a predetermined period of time that satisfies a programmed criteria; detecting any rate of change in respiratory rate; determining whether the change in minute ventilation is a sole function of increased tidal volume; and delivering cardiac pacing therapy if minute ventilation criteria are met at a pacing rate above the intrinsic heart rate for a predetermined period of time. The computer readable medium may also include storing at least one data item related to the step of detecting an increase in minute ventilation.

A cardiac pacing apparatus is disclosed that includes means for detecting a series of intrinsic depolarizations of a heart; means for detecting minute ventilation and respiratory rate; means for sampling baseline minute ventilation and respiratory rate, detecting an increase in minute ventilation over a predetermined period of time that satisfies a programmed criteria, detecting any rate of change in respiratory rate, and determining whether the change in minute ventilation is a sole function of increased tidal volume; and means for delivering cardiac pacing therapy if minute ventilation criteria are met at a pacing rate above the intrinsic heart rate for a predetermined period of time. The cardiac pacing apparatus may also include means for storing at least one data item related to the step of detecting an increase in minute ventilation.

### BRIEF DESCRIPTION OF THE DRAWINGS

The accompanying drawings, which are incorporated in and constitute a part of this specification, exemplify the embodiments of the present invention and, together with the description, serve to explain and illustrate principles of the invention. The drawings are intended to illustrate major features of the exemplary embodiments in a diagrammatic manner. The drawings are not intended to depict every feature of actual embodiments nor relative dimensions of the depicted elements, and are not drawn to scale.
Figure 1 is schematic view illustrating an implantable medical device (IMD) coupled with the heart of a patient through leads introduced into the right atrium and ventricles;
Figure 2 is a schematic view of the IMD in further detail;
Figure 3 is a block diagram illustrating an implantable pacing system;
Figures 4A and 4B are graphical depictions showing heart rate and blood pressure response with and without syncope;
Figures 5A and 5B are graphical depictions showing fold change from baseline with and without syncope;
Figure 6 is a graphical depiction change in minute ventilation from baseline for responders and non-responders;
Figure 7 is a graphical depiction of minute ventilation during syncope; and
Figure 8 is a flow chart of a process for detecting and treating syncope.

### DETAILED DESCRIPTION

A pacing and sensing algorithm which uses respiratory input to detect impending neurocardiogenic syncope is described. This algorithm, when used with conventional pacemakers, may be utilized to prevent recurrent syncope in patients with isolated recurrent neurocardiogenic syncope, as well in patients with established indications for pacemakers (e.g., conduction system disease, sudden cardiac death prophylaxis, cardiac resynchronization) and concurrent neurocardiogenic syncope. The latter group is increasingly prevalent. The algorithm provides both a diagnostic and therapeutic option for patients with isolated neurocardiogenic syncope, and diverse treatment options for patients with an existing need for pacemaker therapy.

The inventors discovered that a 2 to 3 fold increase in minute ventilation precedes the drop in heart rate and blood pressure in neurocardiogenic syncope. This increase in minute ventilation is driven exclusively by increases in tidal volume (TV) rather than respiratory rate, which allows for easy distinction between impending neurocardiogenic syncope and other physiologic causes of increased minute ventilation (for example, exercise, pain, anxiety, primary respiratory distress, and heart failure). The syncope detection method utilizes respiratory input for early detection of syncope, with subsequent triggered pacing to prevent syncope. When the detected minute ventilation exceeds a predetermined threshold value without a corresponding increase in respiratory rate, a predetermined pacing therapy is provided. Neurocardiogenic syncope can also be detected noninvasively using, for example, Holter or event monitors. In these, the neurocardiogenic syncope event is stored.

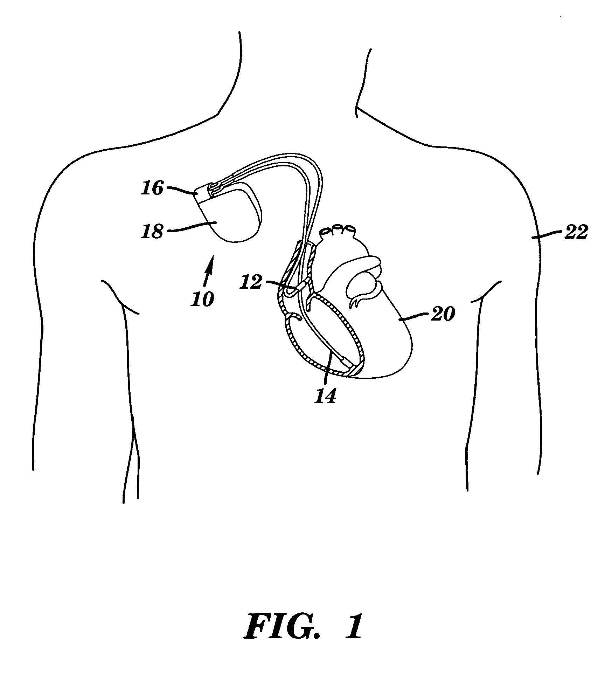

An embodiment of the invention will now be described in detail with reference to Figure 1. Figure 1 is a schematic view of one embodiment of a device that may practice the invention. Implantable medical device (IMD) 10 is a pacemaker having at least one of atrial pacing and sensing lead 12, ventricular pacing and sensing lead 14, or both, attached to connector module 16 of hermetically sealed enclosure 18 and implanted near human or mammalian heart 20 of a patient 22. Pacing and sensing leads 12 and 14 sense electrical signals attendant to the depolarization and repolarization of the heart 20, and further provide pacing pulses for causing depolarization of cardiac tissue in the vicinity of the distal ends thereof. Leads 12 and 14 may have unipolar or bipolar electrodes disposed thereon.

IMD 10 is one example of a device that may be employed to supply therapy to treat syncope. In particular, IMD 10 may deliver pacing pulses to heart 20 to cause heart 20 to beat at a rate faster than the intrinsic rate of heart 20. By maintaining a high heart rate, IMD 10 may maintain high cardiac output when patient 22 experiences syncope, and thereby reduce the risk that patient 22 will faint, i.e., lose consciousness.

The invention is not limited to applications with a dual-chamber pacemaker such as IMD 10, however. The invention may be applied to a variety of external or implantable devices, including multi-chamber pacemakers. Devices other than pacemakers, such as external or implantable drug delivery devices, may also apply the techniques of the invention to detect the onset of syncope and apply therapy to reduce the risk that the patient 22 will faint.

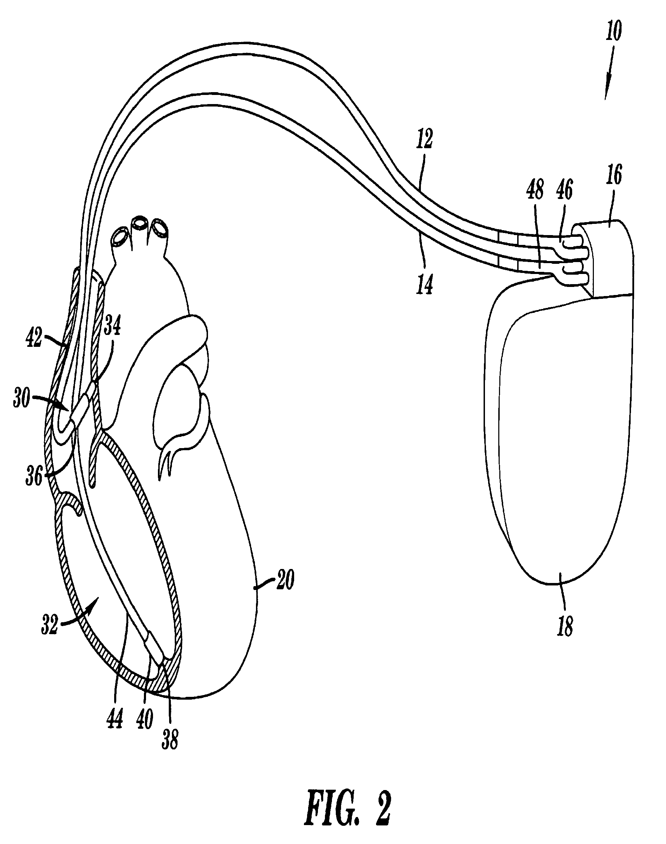

Figure 2 shows IMD 10, with connector module 16 and hermetically sealed enclosure 18 located in and near human or mammalian heart 20. Atrial and ventricular pacing leads 12 and 14 extend from connector module 16 to the right atrium 30 and right ventricle 32, respectively, of heart 20. Atrial electrodes 34 and 36 disposed at the distal end of atrial pacing lead 12 are located in right atrium 30. Ventricular electrodes 38 and 40 disposed at the distal end of ventricular pacing lead 14 are located in right ventricle 32. Pulse generators (not shown in Figure 2) inside enclosure 18 generate pacing pulses. The pacing pulses are delivered to right atrium 30 or right ventricle 32 by electrodes 34, 36, 38, 40.

When addressing a possible episode of syncope, the pacing pulses may maintain a high cardiac output and reduce the risk that patient 22 will faint. Pacing heart 20 to prevent the undesirable effects of syncope is called "preventative pacing." The pacing generators and associated leads and electrodes are an example of a therapy module that can deliver therapy to patient 22 to address syncope.

In addition to pacing, IMD 10 may apply other forms of therapy, which need not be related to treatment of syncope. In Figure 2, for example, atrial lead 12 and ventricular lead 14 include defibrillation electrodes 42 and 44, respectively. Defibrillation electrodes 42 and 44 deliver defibrillation shocks to right atrium 30 or right ventricle 32.

Atrial and ventricular leads 12, 14 each include an elongated insulative lead body carrying one or more conductors insulatively separated from one another. At the proximal end of leads 12, 14 are bifurcated connectors 46, 48, which electrically couple the connectors to connector module 16 of IMD 10.

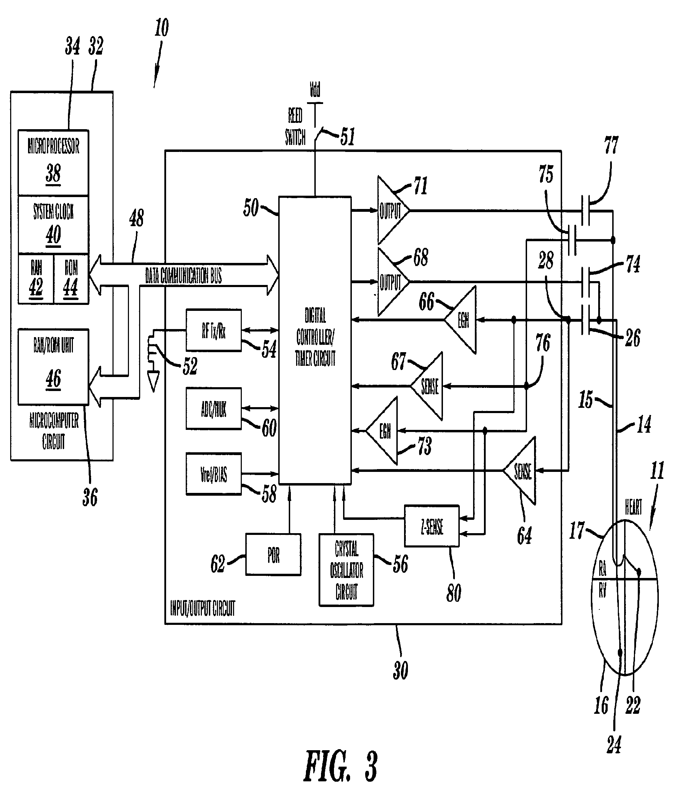

Figure 3 shows a block diagram illustrating the constituent components of IMD 10 in accordance with one embodiment of the invention, in which IMD 10 is a pacemaker having a microprocessor-based architecture. The IMD 10 includes an IPG 13 and leads 14 and 15. The IPG operating system within IPG 13 includes a microprocessor-based microcomputer circuit 32 coupled through a data communications bus 48 with input/output circuit 30. It will be understood by those skilled in the art that the electrical components represented in Figure 3 are powered by an appropriate implantable-grade battery power source (not shown). In one embodiment, pacemaker 10 is capable of operating in various non-rate-responsive modes that include DDD, DDI, WI, VOO, VVT, AAI and AOO, as well as corresponding rate-responsive modes of DDDR, DDIR, WIR, VOOR, VVTR, AAIR and AOOR. Furthermore, pacemaker 10 can be programmably configured to operate such that it varies its rate only in response to one selected sensor output, or in response to both sensor outputs, if desired.

Microcomputer circuit 32 includes an on-board circuit 34 and an off-board circuit 36. On-board circuit 34 includes a microprocessor 38, a system clock 40, and on-board RAM 42 and ROM 44. Off-board circuit 36 includes an off-board RAM/ROM unit 46 providing additional memory. Microcomputer circuit 32 is coupled by data communication bus 48 to a digital controller/timer circuit 50. Microcomputer circuit 32 may be fabricated of custom IC devices augmented by standard RAM/ROM components.

The input/output circuit 30 contains the operating input and output analog circuits for digital controlling and timing circuits necessary for the detection of electrical signals derived from the heart, such as the cardiac electrogram (EGM), output from sensors (not shown) connected to the leads 14 and 15, as well as for the application of stimulating pulses to the heart to control its rate as a function thereof under the control of software-implemented algorithms in microcomputer circuit 32.

An antenna 52 is connected to input/output circuit 30 for purposes of uplink/downlink telemetry through a radio frequency (RF) Transmitter/Receiver Circuit (RF TX/RX) 54. Uplink and downlink telemetry transmission of programming commands and analog and digital data between antenna 52 and an external device, such as an external programmer (not shown), can be accomplished employing any of the hardware and operating systems known in the art. A reed switch 51 is connected to input/output circuit 30 to enable the patient to trigger storage of syncope data when the patient feels fainting symptoms.

A crystal oscillator circuit 56, typically a 32,768 Hz crystal-controlled oscillator, provides main timing clock signals to digital controller/timer circuit 50. A Vref/Bias circuit 58 generates a stable voltage reference and bias currents for the analog circuits of input/output circuit 30. An ADC/multiplexer circuit (ADC/MUX) 60 digitizes analog signals and voltages to provide telemetry and a replacement time-indicating or end-of-life function (EOL). A power-on-reset circuit (POR) 62 functions to initialize the pacemaker 10 with programmed values during power-up, and reset the program values to default states upon the detection of a low battery condition or transiently in the presence of certain undesirable conditions such as unacceptably high electromagnetic interference (EMI), for example.

The operating mode and parameter value can be made programmable and are stored in the microcomputer circuit 32. Commands for controlling the timing of the sensing of sense events and delivery of atrial and ventricular pacing pulses are coupled by bus 48 to digital controller/timer circuit 50. Digital timers of digital controller/timer 30 time out the overall escape interval of the pacemaker, as well as various refractory, blanking and other timing windows for controlling the operation of the peripheral components coupled with leads 14 and 15 within input/output circuit 50. The microcomputer circuit 32 may include computer-readable medium. The term "computer-readable medium" should be taken to include a single medium or multiple media that store one or more sets of instructions. The term "computer-readable medium" shall also be taken to include any medium that is capable of storing, encoding or carrying a set of instructions for execution by the machine and that cause the machine to perform any one or more of the methodologies of the present disclosure. The term "computer-readable medium" shall accordingly be taken to include, but not be limited to, solid-state memories, and optical and magnetic media.

Digital controller/timer circuit 50 is coupled to atrial and ventricular sense amplifiers (SENSE) 64 and 67, respectively, to atrial and ventricular electrogram (EGM) sense amplifiers 66 and 73, to atrial and ventricular pacing pulse generators (OUTPUT) 68 and 71, respectively, and to an impedance measurement circuit (Z-SENSE) 80.

An intracardiac pace/sense electrode 24 located near the distal end of lead 14 and positioned within the right ventricle 16 and a distally located intracardiac pace/sense electrode 22 of atrial lead 15 is positioned within the right atrium 17 are illustrated in Figure 3. Ventricular pace/sense electrode 24 is coupled by a lead conductor of ventricular lead 14 to a connector element of the IPG 13 that is electrically connected with capacitors 26 and 74. Similarly, atrial pace/sense electrode 22 is coupled by a lead conductor of atrial lead 15 to a further connector element of the IPG 13 that is electrically connected with capacitors 75 and 77.

Capacitor 77 couples the atrial lead conductor to the output of the atrial pacing pulse generator 71 so that atrial pacing pulses generated by atrial pacing pulse generator 71 can be delivered to the atrial pace/sense electrode 22. Capacitor 74 couples the ventricular lead conductor to the output of the ventricular pacing pulse generator 68 so that ventricular pacing pulses generated by ventricular pacing pulse generator 71 can be delivered to the ventricular pace/sense electrode 24.

Capacitor 75 couples the atrial lead conductor to node 76 and the input of the atrial sense amplifier 67, the input of atrial EGM amplifier 73 and one input/output of the impedance measurement circuit 80. Capacitor 26 couples the ventricular lead conductor to the input of the ventricular sense amplifier 64, the input of ventricular EGM amplifier 66, and another input/output of the impedance measurement circuit 80. The atrial and ventricular sense amplifiers 67 and 64 amplify and process atrial and ventricular signals picked up from electrodes 22 and 24 to generate sense event signals for re-setting the escape interval and/or AV delay timers within digital controller/timer circuit 50. The EGM signals developed by atrial and ventricular EGM amplifiers 64 and 73 are used in those occasions when the IPG 13 is being interrogated by the external programmer/transceiver (not shown) in order to uplink telemetry transmit a representation of the analog atrial and ventricular EGM to analyze the electrical heart activity.

The impedance measurement circuit 80 operates to continually or periodically measure impedance across the pace/sense electrodes 22 or 24 and the IPG case (or another pair of sense electrodes disposed across the patient's chest) that is modulated by inspiration and exhalation and to develop a breathing rate. By applying a high frequency AC carrier signal between a pair of electrodes disposed about the heart or subcutaneously implanted across the chest, e.g., pace/sense electrodes 22 and 24, a current flows through blood and lungs. Blood is more conductive than air. As volume in the lungs changes it has a large impact on measured impedance. Demodulation and processing can reveal respiratory parameters from the impedance vs. time signal, as described for example, in U.S. Pat. No. 5,318,597. Respiration parameters including respiration rate (RR) in breaths per minute, tidal volume (TV) in liters, and minute ventilation (MV), that is the product of TV and RR, can be derived from the aforementioned respiratory component of the impedance vs. time waveform. Minute ventilation can function as a rate control parameter for a rate adaptive pacemaker because it is a parameter that varies directly with hemodynamic demand.

IMD 10 may includes one or more activity sensors. The activity sensor may include an accelerometer, such as an electrostatic accelerometer, a piezoceramic accelerometer or a microelectromechanical accelerometer, that typically provides a sensor output that varies as a function of a measured parameter relating to a patient's metabolic requirements. In other words, activity sensor detects motion of the patient that accompanies physical activity, and may adjust the pacing rate to the metabolic needs associated with the physical activity. In addition, the activity sensor may be configured to detect a change in the posture of patient. The output of activity sensor is coupled to input/output circuit 30. The rate of heart 20 is controlled by software-implemented algorithms stored within microcomputer circuit 32.

IMD 10 may further include one or more other sensors (not shown) to sense other physiological signals or may receive signals from one or more other sensors that sense other physiological signals. An example of such a sensor is a pressure sensor that responds to a blood pressure such as arterial blood pressure. The pressure sensor may be disposed in a chamber of heart 11, e.g., on the distal end of lead, or may be deployed at another site in or around the cardiovascular system.

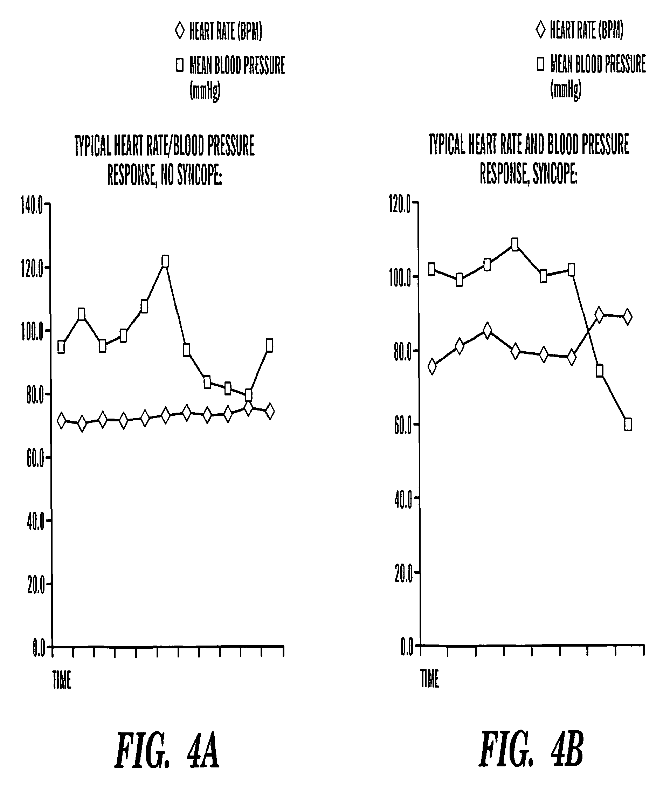

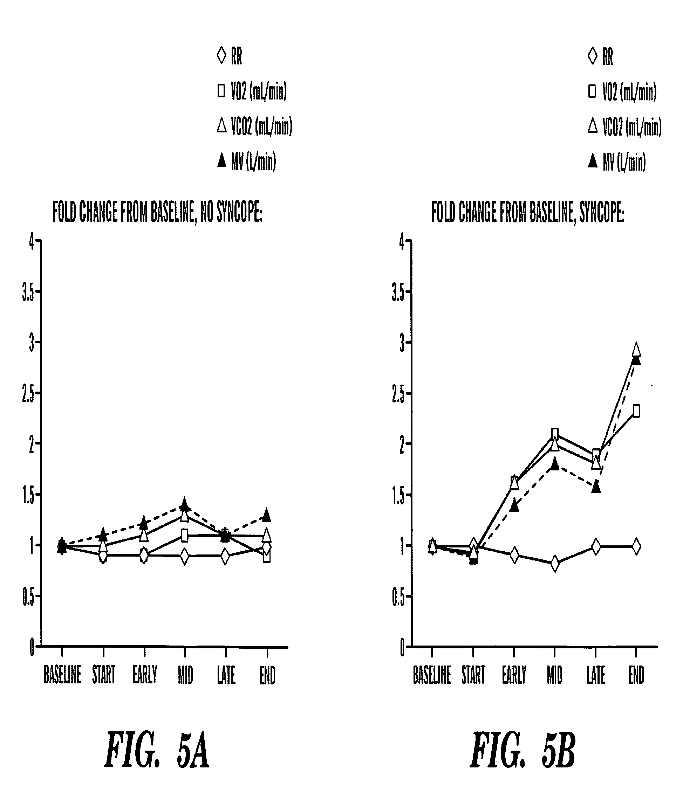

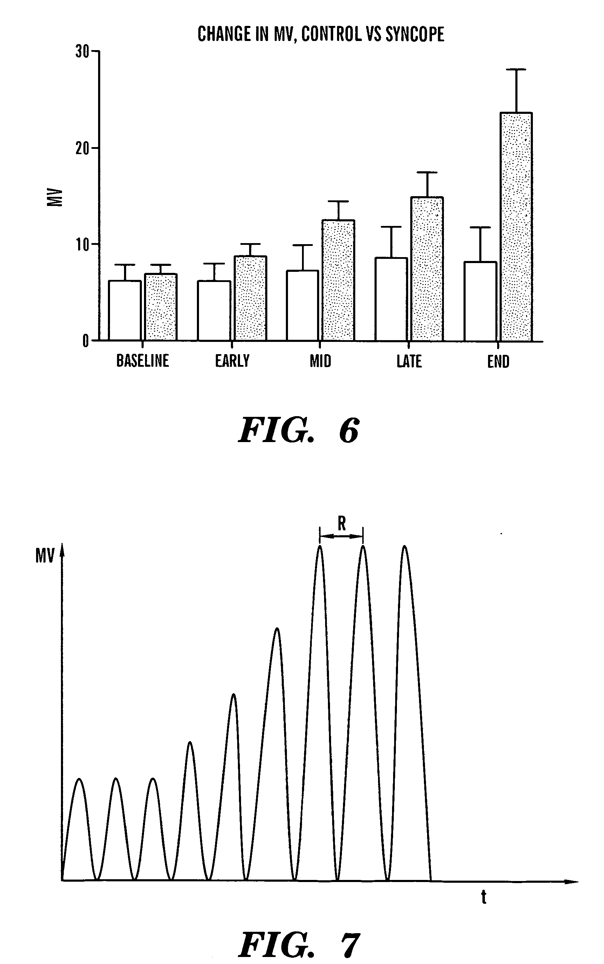

With reference to Figures 4A and 4B, a typical heart rate and blood pressure response is shown in patients without syncope (Figure 4A) and with syncope (Figure 4B). As shown in Figures 4A and 4B, syncope is marked by a sudden drop in heart rate and blood pressure which results in decreased cerebral perfusion and subsequent loss of consciousness and postural tone. As shown in Figures 5A and 5B and Figure 6, during syncope, there is an increase in minute ventilation (MV) and tidal volume (VO2/VCO2) while the respiration rate remains the same.

Figure 7 shows the typical evolution of minute ventilation signal (MV) with respect to time in a patient with neurocardiogenic syncope. Note that the respiratory interval (R) remains fixed, while the tidal volume (TV, or TE) increases substantially. As described above, MV is a signal which may be directly obtained from measuring thoracic impedance. Oscillations in thoracic impedance with respect to time allow for the derivation of TV and R. Although thoracic impedance is the principal method for measuring minute ventilation and respiratory rate in patients with pacemakers, other physiologic sensors or inputs could be used, including sensors which measure blood oxygen saturation or patient acceleration.

The measurement of minute ventilation in cardiac pacemakers is obtained between one of the pacemaker sensing and pacing electrodes (leads) and the casing of the pulse generator. If the implanted device is not a pacemaker, then the measurement may be obtained by placing two electrodes in the rib cage. The impedance is then measured in response to the application of constant current (e.g., approximately 200 mA) at a fixed frequency (e.g., usually 8 Hz). From this, one can determine the respiratory period (R), or respiratory rate (breaths per minute). The tidal volume (TV) is represented by the area under the curve, and the minute ventilation is the product of (RxTV)/min.

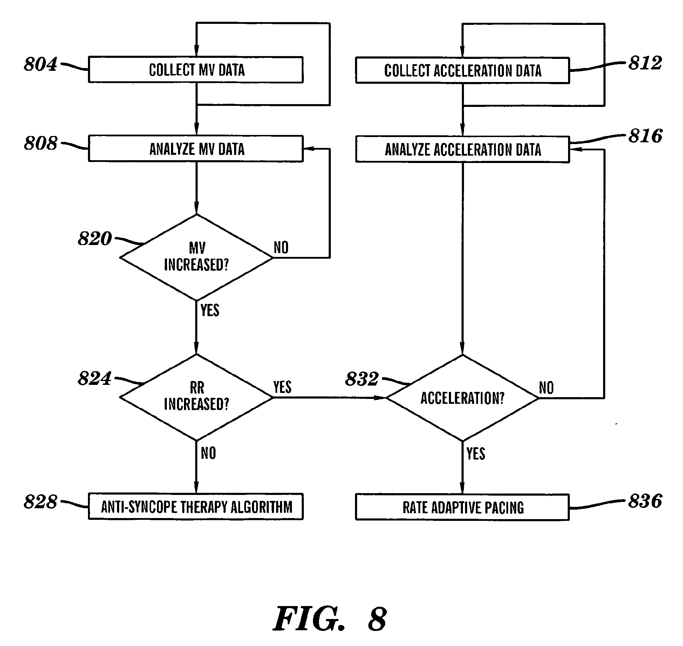

Figure 8 illustrates a process for detecting syncope 800. The process 800 is described in the context of a rate adaptive dual chamber pacemaker with an accelerometer and a process for measuring transthoracic impedance, such as the implantable medical device of Figures 1-3. It will be appreciated that the method may be used with other implantable medical devices or other non-invasive medical devices. In addition, it will be appreciated that the process may vary. The process 800 may include additional steps or fewer steps and the order of the steps may differ.

During baseline conditions, the pacemaker generator and processor collects and analyzes the intrinsic electrical activity of the heart, which is collected via pacing and sensing leads positioned in the right atrium and right ventricle. If the intrinsic electrical activity of the heart is interrupted, the device initiates standard pacing therapies as previously described. In addition, minute ventilation data is collected by continuous measurement of transthoracic impedance as described above (block 804 and block 808). An accelerometer or other sensor also detects patient movement (block 812), which is also analyzed (block 816).

If an increase in minute ventilation is detected (block 820), then the respiratory interval is analyzed (block 824). The respiratory interval may be analyzed over a fixed period of time (e.g., any time period or range of time periods between about 10 seconds and about 5 minutes, including less than ten seconds and more than 5 minutes). If the increased minute ventilation is not accompanied by a decrease in respiratory interval (e.g., the respiratory interval does not decrease for a predetermined period of time, e.g., any time period or range of time periods between about 10 seconds and about 5 minutes, including less than ten seconds and more than 5 minutes), then anti-syncope therapy is initiated (block 828). If, on the other hand, the respiratory interval is decreased, then the accelerometer data is analyzed (block 832). Increased minute ventilation, decreased respiratory interval, and detected acceleration indicate increased physical activity. In this example, the device would function as a typical rate adaptive pacemaker (block 836). Alternatively, increases in minute ventilation, decreased respiratory interval, and no detected acceleration would indicate increased physiologic stress without physical activity (for example, heart failure, anxiety, or respiratory distress); this information may be subsequently used for diagnostic purposes. It will be appreciated that the detection algorithm may be applied without the use of accelerometer data. It will also be appreciated that minute ventilation may be based upon measured MVO2.

The above may be applied to single, dual chamber, biventricular or cardiac resynchronization therapy (CRT) devices, as well as to implantable cardioverter-defibrillators (ICDs). Moreover, the detection algorithm may be applied to any monitoring device which is outfitted to measure intrinsic cardiac electrical activity, minute ventilation, and acceleration. It will be appreciated that the algorithm may be stored in associated device programmers and transtelephonic monitoring devices for the pacemakers or other medical devices. Other exemplary devices that may include the detection algorithm include, for example, implantable loop recorders and transcutaneous heart rhythm monitoring systems (e.g., Holter monitors and event monitors). Application of minute ventilation measurement into implantable loop recorders or event monitors may allow the distinction between neurocardiogenic syncope/vasodepressor syncope from other bradyarrhythmias

Exemplary advantages of embodiments of the invention include, for example, implementation of pacemaker therapy for patients with recurrent neurocardiogenic syncope, a more diverse array of pacing options for patients with known indications for pacemaker therapy, an increase in the overall physiologic input to the pacemaker, which can provide the treating physician with diagnostic information, and integration of minute ventilation, respiratory rate, and heart rate, which may be used in "traditional" pacemaker patients to provide more physiologic pacing.

It should be understood that processes and techniques described herein are not inherently related to any particular apparatus and may be implemented by any suitable combination of components. Further, various types of general purpose devices may be used in accordance with the teachings described herein. It may also prove advantageous to construct specialized apparatus to perform the method steps described herein. The present invention has been described in relation to particular examples, which are intended in all respects to be illustrative rather than restrictive. Those skilled in the art will appreciate that many different combinations of hardware, software, and firmware will be suitable for practicing the present invention.

Moreover, other implementations of the invention will be apparent to those skilled in the art from consideration of the specification and practice of the invention disclosed herein. Various aspects and/or components of the described embodiments may be used singly or in any combination. It is intended that the specification and examples be considered as exemplary only, with the invention being defined by the following claims.

## Claims

1. A cardiac pacing apparatus (10), comprising:
a means (12, 14) for detecting a series of intrinsic depolarizations of a heart;
a means for detecting minute ventilation and respiratory rate;
a means for detecting a change in minute ventilation and respiratory rate over a fixed period of time; and
a means (34, 36, 38, 40) for delivering cardiac pacing therapy when the means for detecting a change in minute ventilation and respiratory rate detects an increase in minute ventilation without a corresponding increase in respiratory rate for a predetermined period of time,
wherein the apparatus is configured to determine whether an increase in minute ventilation is the result of increased tidal volume, with a relatively fixed respiratory rate, which is defined as a less than 25% variance in respiratory period, R, and,
wherein the increase in tidal volume is defined as being more than a 75% increase from baseline.

2. The cardiac pacing apparatus according to claim 1, further comprising:
a means for sampling baseline minute ventilation and respiratory rate, a means for detecting an increase in minute ventilation over a predetermined period of time that satisfies a programmed criteria, a means for detecting any rate of change in respiratory rate, and for determining whether the change in minute ventilation is a sole function of increased tidal volume.

3. The cardiac pacing apparatus according to any of claims 1 - 2, further comprising a means for delivering cardiac pacing therapy, if minute ventilation criteria are met, at a pacing rate above intrinsic heart rate for a predetermined period of time.

4. The cardiac pacing apparatus according to any of claims 1 - 3, further comprising a means for storing at least one data item operationally connected to the means for detecting minute ventilation.

5. The cardiac pacing apparatus according to any of claims 1 - 4, wherein the apparatus is adapted to treat an individual suffering from neurocadiogenic syndrome based upon changes in respiratory rate over a predetermined time.

6. The cardiac pacing apparatus of any of claims 1 - 5 comprising an implantable medical device.

7. The cardiac pacing apparatus of claim 6, wherein the implantable medical device is a pacemaker having a microprocessor-based architecture.

8. The cardiac pacing apparatus of claim 7, wherein the pacemaker is programmably configured to operate such that it varies its rate only in response to one or more selected sensor outputs.

9. The cardiac pacing apparatus of any of claims 1 - 8 comprising an external or implantable drug delivery device.

## Patentansprüche

1. Herzschrittmachervorrichtung (10), welche Folgendes aufweist:
Mittel (12, 14) zum Detektieren bzw. Nachweisen einer Reihe intrinsischer Depolarisationen eines Herzens;
Mittel zum Detektieren des Atemminutenvolumens und der Atemfrequenz;
Mittel zum Detektieren einer Veränderung des Atemminutenvolumens und der Atemfrequenz über einen festgesetzten Zeitraum; und
Mittel (34, 36, 38, 40) zum Vorsehen einer kardialen Schrittmachertherapie, wenn das Mittel zum Nachweis einer Veränderung des Atemminutenvolumens und der Atemfrequenz einen Anstieg des Atemminutenvolumens ohne einen entsprechenden Anstieg der Atemfrequenz für einen vorbestimmte Zeitraum detektiert,
wobei die Vorrichtung konfiguriert ist, zu bestimmen, ob ein Anstieg des Atemminutenvolumens das Ergebnis eines erhöhten Tidal- bzw. Atemzugvolumens mit einer relativ festen Atemfrequenz ist, welche definiert ist als eine Abweichung von weniger als 25% innerhalb der Atmungsperiode R, und
wobei die Zunahme des Atemzugvolumens definiert ist als ein Anstieg von mehr als 75% von Basis- bzw. Ausgangslinie.

2. Herzschrittmachervorrichtung nach Anspruch 1, welche ferner Folgendes aufweist: Mittel zur Probenahme des Ausgangs-Atemminutenvolumens und der Ausgangs-Atemfrequenz, Mittel zum Detektieren eines Anstiegs des Atemminutenvolumens über einen vorbestimmten Zeitraum, der einem programmierten Kriterium genügt, Mittel zum Detektieren jeder Veränderung der Atemfrequenz und zum Bestimmen, ob die Veränderung des Atemminutenvolumens ausschließliche eine Funktion des erhöhten Atemzugvolumens ist.

3. Herzschrittmachervorrichtung nach einem der Ansprüche 1-2, welche ferner Mittel aufweist zum Vorsehen einer kardialen Schrittmachertherapie, wenn Atemminutenvolumen-Kriterien eingehalten sind, und zwar bei einer Schrittmacherfrequenz oberhalb der intrinsischen Herzfrequenz für einen festgelegten Zeitraum.

4. Herzschrittmachervorrichtung nach einem der Ansprüche 1-3, welche ferner ein Mittel zum Speichern von mindestens einem Datensatz aufweist, welches betriebsmäßig mit dem Mittel zum Detektieren des Atemminutenvolumens verbunden ist.

5. Herzschrittmachervorrichtung nach einem der Ansprüche 1-4, wobei die Vorrichtung geeignet ist, um einen Patienten zu behandeln, der an einem neurokardiogenen Syndrom bzw. neurokardiogenen Synkopen leidet, und zwar basierend auf den Veränderungen der Atemfrequenz über einen vorbestimmten Zeitraum.

6. Herzschrittmachervorrichtung nach einem der Ansprüche 1-5, die ein implantierbares medizinisches Gerät aufweist.

7. Herzschrittmachervorrichtung nach Anspruch 6, wobei das implantierbare medizinische Gerät ein Schrittmacher ist, welcher eine auf einem Mikroprozessor basierte Bauweise hat.

8. Herzschrittmachervorrichtung nach Anspruch 7, wobei der Schrittmacher programmierbar konfiguriert ist, um derart zu arbeiten, dass er seine Frequenz nur ansprechend auf eine oder mehrere ausgewählte Sensorausgabewerte ändert.

9. Herzschrittmachervorrichtung nach einem der Ansprüche 1-8, welche eine externe oder implantierbare Medikamentenabgabevorrichtung aufweist.

## Revendications

1. Appareil de stimulation cardiaque (10), comprenant:
des moyens (12, 14) pour détecter une série de dépolarisations intrinsèques d'un coeur;
des moyens pour détecter une ventilation minute et une fréquence respiratoire ;
des moyens pour détecter un changement dans la ventilation minute et la fréquence respiratoire sur une période de temps fixe ;
des moyens (34, 36, 38, 40) pour délivrer une thérapie de stimulation cardiaque lorsque les moyens pour détecter un changement dans la ventilation minute et la fréquence respiratoire détectent une augmentation de la ventilation minute sans une augmentation correspondante de la fréquence respiratoire pendant une période de temps prédéterminée,
dans lequel l'appareil est agencé pour déterminer si une augmentation de la ventilation minute est le résultat d'une augmentation du volume courant, avec une fréquence respiratoire relativement fixe, qui est définie comme une variance inférieure à 25 % de la période respiratoire, R, et
dans lequel l'augmentation du volume courant est définie comme étant une augmentation de plus de 75 % par rapport à une ligne de base.

2. Appareil de stimulation cardiaque selon la revendication 1, comprenant en outre:
des moyens pour échantillonner la ventilation minute et la fréquence respiratoire de la ligne de base, des moyens pour détecter une augmentation de la ventilation minute sur une période de temps prédéterminée qui satisfait un critère programmé, des moyens pour détecter un quelconque taux de variation dans la fréquence respiratoire, et pour déterminer si la variation de la ventilation minute est fonction uniquement de l'augmentation du volume courant.

3. Appareil de stimulation cardiaque selon l'une quelconque des revendications 1 à 2, comprenant en outre des moyens pour délivrer une thérapie de stimulation cardiaque, si les critères de ventilation minute sont satisfaits, à un rythme de stimulation supérieur au rythme cardiaque intrinsèque pendant une période de temps prédéterminée.

4. Appareil de stimulation cardiaque selon l'une quelconque des revendications 1 à 3, comprenant en outre des moyens pour mémoriser au moins un élément de donnée, connectés fonctionnellement aux moyens pour détecter la ventilation minute.

5. Appareil de stimulation cardiaque selon l'une quelconque des revendications 1 à 4, dans lequel l'appareil est adapté à traiter un individu souffrant d'un syndrome neurocardiogénique à l'occurrence de changements dans la fréquence respiratoire pendant un temps prédéterminé.

6. Appareil de stimulation cardiaque selon l'une quelconque des revendications 1 à 5, comprenant un dispositif médical implantable.

7. Appareil de stimulation cardiaque selon la revendication 6, dans lequel le dispositif médical implantable est un stimulateur cardiaque ayant une architecture à base de microprocesseur.

8. Appareil de stimulation cardiaque selon la revendication 7, dans lequel le stimulateur cardiaque est configuré de façon programmable pour opérer de telle sorte qu'il fasse varier sa fréquence seulement en réponse à une ou plusieurs sorties de capteur sélectionnées.

9. Appareil de stimulation cardiaque l'une quelconque des revendications 1 à 8, comprenant un dispositif externe ou implantable de fourniture de médicament.
